# EUROPEAN PATENT APPLICATION

(11) **EP 4 670 614 A1**
(43) Date of publication of application: **31.12.2025**
(21) Application number: 24760415.0
(22) Date of filing: 21.02.2024
(51) Int. Cl.: A61B 1/045, G06T 7/00

(54) **ENDOSCOPE DIAGNOSIS PROGRAM, ENDOSCOPE DIAGNOSIS DEVICE, CONTROL METHOD FOR ENDOSCOPE DIAGNOSIS DEVICE, AND PROGRAM FOR GENERATING ENDOSCOPE DIAGNOSIS TRAINED MODEL**

(30) Priority: 24.02.2023 JP 2023027308
(71) Applicant: National University Corporation Asahikawa Medical University, Asahikawa-shi, Hokkaido 078-8510 (JP); Suncreer Co., Ltd., Sapporo-shi, Hokkaido 060-0012 (JP)
(72) Inventor: FUJIYA Mikihiro, Asahikawa-shi, Hokkaido 078-8510 (JP); MORIICHI Kentaro, Asahikawa-shi, Hokkaido 078-8510 (JP); ANDO Katsuyoshi, Asahikawa-shi, Hokkaido 078-8510 (JP); SATO Hiroki, Asahikawa-shi, Hokkaido 078-8510 (JP); ENOMOTO Katsuaki, Asahikawa-shi, Hokkaido 078-8510 (JP); MORI Masato, Sapporo-shi, Hokkaido 060-0012 (JP); MORI Yuto, Sapporo-shi, Hokkaido 060-0012 (JP)
(74) Representative: Betten & Resch
(86) International application number: PCT/JP2024/006367
(87) International publication number: WO 2024/177117

(57) **Abstract**

[Problem] To provide an endoscope diagnosis program, an endoscope diagnosis device, a control method for an endoscope diagnosis device, and a program for generating an endoscope diagnosis trained model which each make it possible to use endoscope images that have been accumulated to date to diagnose the presence or absence of a lesion, without limitations on the type of endoscope device.

[Solution] A computer is made to function as: a diagnostic-use image acquisition unit 66 that acquires a diagnostic-use image of a site that is subject to diagnosis which has been captured with an endoscope device 20; a color tone correction unit 67 that performs color tone correction on the diagnostic-use image in accordance with a reference color tone, which is a color tone of an image of a site subject to diagnosis having been captured in advance with the endoscope device; and a lesion presence/absence diagnosis unit 68 that inputs the corrected diagnostic-use image into a trained model and that performs a process for diagnosing the presence or absence of a lesion from a result output by the trained model, said trained model having being generated by machine learning of a plurality of training images which have been subjected to color tone correction with the reference color tone.

## Description

### TECHNICAL FIELD

The present invention relates to an endoscope diagnosis program, an endoscope diagnosis device, an endoscope diagnosis device-control method, and a trained model generation program for endoscope diagnosis, each for using an endoscope image captured with an endoscope device to diagnose the presence or absence of a lesion.

### BACKGROUND ART

Ulcerative colitis is a disease in which chronic inflammation occurs in the mucosa of the large intestine, and is one of difficult diseases in which symptoms such as fever, abdominal pain, diarrhea, and blood in feces (blood is mixed in feces) appear when the disease is developed. When the symptoms become severe, various symptoms such as fever, weight loss, and anemia are caused in the whole body. Therefore, it is necessary to perform early detection and appropriate treatment.

For the diagnosis of ulcerative colitis, an endoscope device is generally used. A doctor irradiates a diagnosis target site with illumination light, and diagnoses the presence or absence of inflammation or ulcer by viewing an endoscope image captured with an endoscope device. However, such visual diagnosis is greatly influenced by the skill and experience of the doctor. In addition, it is difficult for even a specialist to make accurate diagnosis because early ulcerative colitis cannot be distinguished from normal tissue, and inflammation includes other types of infectious enteritis such as Campylobacter enterocolitis.

Therefore, development of devices that enable more accurate diagnosis using an endoscope image is underway. For example, JP 2022-60540 A proposes an endoscope device capable of irradiating a diagnosis target site with specific spectral light (so-called narrow band light) and identifying the presence or absence, type, degree of progression, and the like of a lesion using a captured image (Patent Literature 1).

### CITATIONS LIST

### PATENT LITERATURE

Patent Literature 1: JP 2022-60540 A

### SUMMARY OF INVENTION

### TECHNICAL PROBLEMS

However, in the invention described in Patent Literature 1, since specific spectral light is used, the device is expensive and is not widely used in general.

Meanwhile, an endoscope device capable of capturing with illumination light (for example, white light), which is not limited to a specific spectrum, is generally widely used. Therefore, a large amount of endoscope images captured with light that is not limited to a specific spectrum, particularly white light, have been accumulated so far.

In addition, in recent years, with the progress of AI technology, it is possible to detect an abnormality or the like in an image through machine training of images. Therefore, it is expected to develop a device capable of automatically diagnosing the presence or absence of a lesion or the like by causing the endoscope images accumulated so far to undergo machine training.

However, the endoscope image has a different color tone and image quality depending on the device type that captured the image. Therefore, although a large amount of endoscope images have been accumulated, highly accurate diagnosis has not been possible even when machine training is performed using all images taken by different endoscope devices.

It is also possible to perform machine training based on each device type, but the number of images to be machine-trained would be reduced. In addition, machine training must be performed again each time an endoscope device is replaced, and high accuracy cannot be expected immediately after replacement because the number of images is small. Moreover, by the time a sufficient number of images have been accumulated and used for machine training, it will already be time to replace the current endoscope device with a new one, resulting in a vicious cycle of having to start the process all over again.

The present invention has been made to solve the above problems, and an object of the present invention is to provide an endoscope diagnosis program, an endoscope diagnosis device, an endoscope diagnosis device-control method, and a trained model generation program for endoscope diagnosis, which each make it possible to use endoscope images accumulated so far to diagnose the presence or absence of a lesion, without limiting the device type of the endoscope device.

### SOLUTIONS TO PROBLEMS

An endoscope diagnosis program according to the present invention is provided in order to solve the problem of enabling images captured with different endoscope devices to be used to diagnose presence or absence of a lesion. The program diagnoses the presence or absence of a lesion on the basis of an endoscope image captured with an endoscope device, the endoscope diagnosis program causing a computer to function as: a diagnostic-use image acquisition unit that acquires a diagnostic-use image of a diagnosis target site captured with an endoscope device; a color tone correction unit that performs color tone correction on the diagnostic-use image in accordance with a reference color tone that is a color tone of an image of a diagnosis target site captured in advance with the endoscope device; and a lesion presence/absence diagnosis unit that inputs the corrected diagnostic-use image into a trained model and that performs diagnosis processing of the presence or absence of a lesion based on the resulting output, the trained model having been generated through machine training of a plurality of training images that have been subjected to color tone correction in accordance with the reference color tone.

Further, as one aspect of the present invention, in order to solve the problem of further enhancing the diagnosis accuracy by setting the image quality between the images within a certain range, the color tone correction unit may perform the color tone correction and image quality enhancement processing on the diagnostic-use image, and the lesion presence/absence diagnosis unit may perform diagnosis processing of the presence or absence of a lesion by the trained model that has been generated through machine training of the training images that have undergone the same color correction and image quality enhancement processing as performed by the color tone correction unit.

Furthermore, as one aspect of the present invention, in order to solve the problem of enabling a large amount of accumulated images in which no lesion is captured to be used to diagnose the presence or absence of a lesion, the lesion presence/absence diagnosis unit may perform, as an image abnormality detection algorithm, inputting the corrected diagnostic-use image into a trained model that has been generated through machine training of a plurality of the training images corrected in accordance with the reference color tone and in which no lesion is captured, and diagnosing the presence of a lesion when an abnormality in the diagnostic-use image is detected as the resulting output.

In addition, as one aspect of the present invention, in order to solve the problem of enabling images accumulated regardless of the presence or absence of the captured lesion to be used to diagnose the presence or absence of a lesion, the lesion presence/absence diagnosis unit may perform, as an algorithm for detecting an image feature amount, inputting the corrected diagnostic-use image into a trained model that has been generated through machine training of a plurality of the training images corrected in accordance with the reference color tone and in which no lesion is captured and a plurality of the training images corrected in accordance with the reference color tone and in which lesions are captured, and diagnosing the presence of a lesion when the feature amount of the diagnostic-use image is determined to be a predetermined value or more as the resulting output, and diagnosing the absence of a lesion when the feature amount of the diagnostic-use image is determined to be less than the predetermined value as the resulting output.

An endoscope diagnosis device according to the present invention is provided in order to solve the problem of enabling images captured with different endoscope devices to be used to diagnose presence or absence of a lesion. The device diagnoses the presence or absence of a lesion on the basis of an endoscope image captured with an endoscope device, the device including: a diagnostic-use image acquisition unit that acquires a diagnostic-use image of a diagnosis target site captured with an endoscope device; a color tone correction unit that performs color tone correction on the diagnostic-use image in accordance with a reference color tone that is a color tone of an image of a diagnosis target site captured in advance with the endoscope device; and a lesion presence/absence diagnosis unit that inputs the corrected diagnostic-use image into a trained model and that performs diagnosis processing of the presence or absence of a lesion based on the resulting output, the trained model having been generated through machine training of a plurality of training images that have been subjected to color tone correction in accordance with the reference color tone.

An endoscope diagnosis device-control method according to the present invention is provided in order to solve the problem of enabling images captured with different endoscope devices to be used to diagnose the presence or absence of a lesion. The method diagnoses presence or absence of a lesion on the basis of an endoscope image captured with an endoscope device, the method including: a diagnostic-use image acquisition step of acquiring a diagnostic-use image of a diagnosis target site captured with an endoscope device; a color tone correction step of performing color tone correction on the diagnostic-use image in accordance with a reference color tone that is a color tone of an image of a diagnosis target site captured in advance with the endoscope device; and a lesion presence/absence diagnosis step of inputting the corrected diagnostic-use image into a trained model and performing diagnosis processing of the presence or absence of a lesion based on the resulting output, the trained model having been generated through machine training of a plurality of training images that have been subjected to color tone correction in accordance with the reference color tone.

A trained model generation program for endoscope diagnosis according to the present invention is provided in order to solve the problem of generating a trained model that can use images captured with different endoscope devices to diagnose presence or absence of a lesion. The program generates a trained model to be used in endoscope diagnosis for diagnosing the presence or absence of a lesion on the basis of an endoscope image captured with an endoscope device, the trained model generation program causing a computer to function as: a training image acquisition unit that acquires a plurality of training images of a diagnosis target site captured with an endoscope device; a color tone correction unit that performs color tone correction on each of the training images in accordance with a reference color tone that is a color tone of an image of a diagnosis target site captured in advance with the endoscope device; and a trained model generation unit that generates a plurality of trained models using a machine training algorithm; and a trained model determination unit that inputs a validation image in which presence or absence of a lesion has been validated to each of the generated trained models and that determines the trained model having a highest correct answer rate as a trained model to be used for endoscope diagnosis.

As one aspect of the present invention, in order to solve the problem of improving the diagnosis accuracy in the case of using the training image in which a lesion is captured, the color tone correction unit may execute color tone correction in accordance with the reference color tone in an area other than the lesion existing area when the lesion is captured in the training image.

Furthermore, as one aspect of the present invention, in order to solve the problem of increasing the number of training images in a pseudo manner and further improving the diagnosis accuracy, a computer may be made to function as an affine transformation unit that performs an arbitrary affine transformation on the training image corrected by the color tone correction unit, and the trained model generation unit may cause machine training to be performed on the training image transformed by the affine transformation unit together with the training image corrected by the color tone correction unit to generate a trained model.

### ADVANTAGEOUS EFFECTS OF INVENTION

According to the present invention, it is possible to use endoscope images accumulated so far to diagnose the presence or absence of a lesion, without limiting the device type of the endoscope device.

### BRIEF DESCRIPTION OF DRAWINGS

Fig. 1 is a block diagram illustrating a first embodiment of an endoscope diagnosis device according to the present invention.
Fig. 2 is a flowchart illustrating a processing flow of a trained model generation program for endoscope diagnosis according to the first embodiment.
Fig. 3 is a flowchart illustrating a processing flow of an endoscope diagnosis program according to the first embodiment.
Fig. 4 is a schematic diagram illustrating a combination of training images (training data) and test data of stratified 5-fold cross-validation used for generation of a trained model and validation of diagnosis accuracy in Example 1.

### DESCRIPTION OF EMBODIMENTS

Hereinafter, a first embodiment of an endoscope diagnosis program, an endoscope diagnosis device, an endoscope diagnosis device-control method, and a trained model generation program for endoscope diagnosis according to the present invention will be described with reference to the drawings.

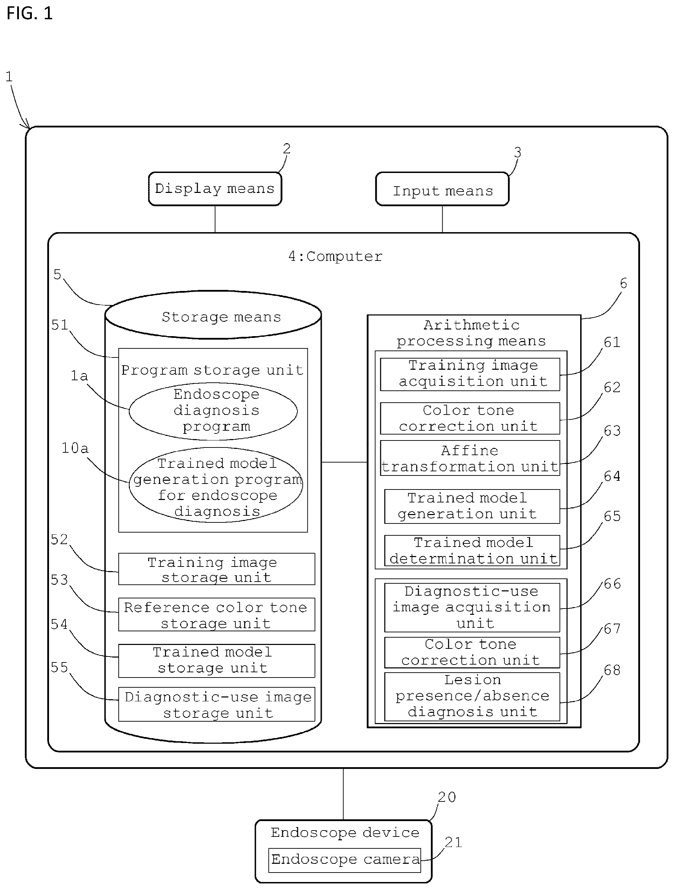

The endoscope diagnosis device 1 automatically diagnoses the presence or absence of a lesion by inputting an endoscope image captured with the endoscope device 20. In the first embodiment, as illustrated in Fig. 1, the endoscope diagnosis device 1 mainly includes a display means 2, an input means 3, and a computer 4 including a storage means 5 and an arithmetic processing means 6. The endoscope diagnosis device 1 is connected to the endoscope device 20 in a state of being able to receive image data.

The endoscope diagnosis device 1 of the first embodiment also has a function of generating a trained model for endoscope diagnosis by a trained model generation program for endoscope diagnosis 10a.

Furthermore, the endoscope device 20 is a device including a general medical endoscope camera 21, and is connected so as to be able to output an endoscope image captured with illumination light (for example, white light) and transmit the endoscope image to the endoscope diagnosis device 1.

Note that, in the first embodiment, the endoscope diagnosis device 1 and the endoscope device 20 are configured as separate devices, but the endoscope diagnosis device 1 and the endoscope device 20 may be configured as an integrated device.

Hereinafter, each configuration of the endoscope diagnosis device 1 will be described in detail.

The display means 2 includes a liquid crystal display or the like, and displays various types of information to the user. For example, the display means 2 displays an input image for inputting calculation conditions or the like, a diagnosis result diagnosed by arithmetic processing, or the like.

The input means 3 includes a mouse, a keyboard, and the like, and inputs various selections and instructions by the user, and is used to input calculation conditions and the like in the present embodiment.

In the first embodiment, the display means 2 and the input means 3 are separately provided, but the present invention is not limited to this configuration, and may be configured by a display input means having both a display function and an input function like a touch panel.

The computer 4 is a device for executing the endoscope diagnosis program 1a and a trained model generation program for endoscope diagnosis 10a, and in the present embodiment, as illustrated in Fig. 1, includes a storage means 5 and an arithmetic processing means 6. Although not illustrated, the computer 4 includes a power supply device that supplies power to the arithmetic processing means 6 and the like, and a communication means such as a local area network (LAN), wireless fidelity (Wi-Fi), or Bluetooth.

The storage means 5 stores various data and functions as a working area when the arithmetic processing means 6 performs arithmetic processing. In the present embodiment, the storage means 5 includes a hard disk, a read only memory (ROM), a random access memory (RAM), a flash memory, and the like, and as illustrated in Fig. 1, includes a program storage unit 51, a training image storage unit 52 that stores a training image used for machine training, a reference color tone storage unit 53 that stores a reference color tone used for color tone correction, a trained model storage unit 54 that stores a trained model generated by the trained model generation program for endoscope diagnosis 10a, and a diagnostic-use image storage unit 55 that stores a diagnostic-use image used for diagnosis.

In the program storage unit 51, an endoscope diagnosis program 1a for acquiring an endoscope image captured with the endoscope device 20 and diagnosing the presence or absence of a lesion and the trained model generation program for endoscope diagnosis 10a for generating a trained model to be used in the endoscope diagnosis program 1a are installed. Then, the arithmetic processing means 6 executes the endoscope diagnosis program 1a and the trained model generation program for endoscope diagnosis 10a to cause the computer 4 to function as each component described later.

Note that the use form of the endoscope diagnosis program 1a and the trained model generation program for endoscope diagnosis 10a is not limited to the above configuration. For example, the endoscope diagnosis program 1a and the trained model generation program for endoscope diagnosis 10a may be stored in a non-transitory recording medium readable by the computer 4, such as a CD-ROM or a DVD-ROM, and may be directly read from the recording medium to be executed. In addition, the endoscope diagnosis program 1a and the trained model generation program for endoscope diagnosis 10a may be used from an external server or the like by a cloud computing method or an application service provider (ASP) method.

The training image storage unit 52 is a storage area that stores training images for generating a trained model for endoscope diagnosis by the trained model generation program for endoscope diagnosis 10a. In the first embodiment, a plurality of training images (raw images) that have been captured and accumulated so far with various types of illumination light including white light with a plurality of types of endoscope devices, a corrected training image (color tone corrected image) corrected by the color tone correction unit 62 described later, and a training image (affine transformation image) obtained by affine transformation of the corrected training image are stored.

In addition, for the training images in the first embodiment, images of a diagnosis target site captured with a plurality of types of endoscope devices are used, the images being confirmed in advance that no lesion is captured. The illumination light used when the training image is captured is not particularly limited, but in the first embodiment, an image captured with so-called white light, which is not limited to specific spectral light, is used.

The reference color tone storage unit 53 is a storage area that stores a reference color tone to be a reference for color tone correction. The reference color tone in the first embodiment is a color distribution based on RGB values or the like obtained by analyzing an image captured in advance by an arbitrary endoscope device. An existing program code or an original program code can be used to acquire the color distribution. As the existing program code, a technology based on Hue analysis, K-means clustering, Color histograms, Convolutional neural networks, Principal component analysis, Self-organizing maps, Gaussian Mixture Models, Support Vector Machines, Decision Trees, OpenCV, matplotlib, or the like can be used. In addition, a hue analysis tool in image processing software can also be used.

The reference color tone is not limited to the color distribution based on RGB values, but numerical values based on color gradation, brightness (contrast), intensity (brightness), and the like can also be used.

The trained model storage unit 54 is a storage area for storing the trained model generated by the trained model generation program for endoscope diagnosis 10a. In the first embodiment, a plurality of trained models (candidate models) generated by the trained model generation unit 64 and a trained model (diagnosis model) to be used for endoscope diagnosis determined by the trained model determination unit 65 are stored.

The diagnostic-use image storage unit 55 is a storage area that stores a diagnostic-use image intended for the diagnosis of lesion presence or absence. In the first embodiment, the diagnostic-use image (raw image) acquired from the endoscope device 20 and the corrected diagnostic-use image (color tone corrected image) corrected by the color tone correction unit 67 are stored. The diagnostic-use image (raw image) acquired from the endoscope device 20 may be an image captured in real time with the endoscope camera 21 or an image captured in advance with the endoscope camera 21. In addition, the illumination light used when the diagnostic-use image is captured is not particularly limited, but in the first embodiment, an image captured with so-called white light, which is not limited to specific spectral light, is used.

The arithmetic processing means 6 includes a central processing unit (CPU) or the like, and executes the trained model generation program for endoscope diagnosis 10a installed in the program storage unit 51 to function as a training image acquisition unit 61, the color tone correction unit 62, an affine transformation unit 63, the trained model generation unit 64, and the trained model determination unit 65, and executes the endoscope diagnosis program 1a to function as a diagnostic-use image acquisition unit 66, the color tone correction unit 67, and a lesion presence/absence diagnosis unit 68.

The training image acquisition unit 61 acquires a plurality of training images of the diagnosis target site captured with the endoscope device. In the first embodiment, the training image (raw image), the corrected training image (color tone corrected image), and the training image after affine transformation (affine transformation image) stored in the training image storage unit 52 are appropriately acquired.

The color tone correction unit 62 performs color tone correction on the training images (raw images) accumulated so far and acquired by the training image acquisition unit 61, and stores the corrected training images (color tone corrected images) in the training image storage unit 52.

The color tone correction unit 62 according to the first embodiment acquires the reference color tone from the reference color tone storage unit 53, corrects the color tone of each of the training images in accordance with the reference color tone, and performs image quality enhancement processing on each of the training images.

The color tone correction is performed, for example, by applying the color distribution stored as the reference color tone to the approximate color of the training image. By performing such color tone correction, the training images are smoothed, and images with different color tones and image qualities depending on the device type can be learned together. As a result, the images accumulated so far can be effectively used. For such color tone correction, an existing program code or an original program code can be used. As the existing program code, Histogram Equalization, Retinex Algorithm, Deep Learning Networks, Color Transfer, Automatic white balance, Gamma Correction, Color Space Conversion, Style Transfer, and the like can be used. In addition, a hue correction tool in image processing software can also be used.

Furthermore, in the image quality enhancement processing, for example, noise removal, image smoothing, blurring removal, mosquito noise correction, sharpening, improvement of similar colors, chromatic aberration correction, enlargement, and the like are executed. By such image quality enhancement processing, generation of the trained model can be accelerated (convergence can be promoted), and diagnosis accuracy using the trained model generated by unifying image quality between images can also be improved. For the image quality enhancement processing, an existing program code or an original program code can be used. As the existing program code, ESRGAN, CUGAN, Cycle GAN, TecoGAN, pix2pix, Super-Resolution Convolutional Neural Network, SRGAN, CodeFormer, and the like can be used. Furthermore, a noise processing tool, a sharpening tool, or the like in image processing software can also be used.

Note that the order of execution of the color tone correction and the image quality enhancement processing by the color tone correction unit 62 is not limited, and any one may be executed first.

The affine transformation unit 63 acquires the corrected training image from the training image storage unit 52, executes affine transformation, and causes the training image storage unit 52 to store the training image after affine transformation as a training image different from the training image before affine transformation.

The affine transformation is to transform an image such as rotation, enlargement/reduction, parallel movement, or skew. This allows for the pseudo-generation of images with varying shooting angles, as well as different distances and positions between the distal end of the endoscope camera and the diagnosis target site, without the need for actual image capture. In addition, by storing the image as a different image, the number of images to be machine-trained can be increased, and the diagnosis accuracy of the trained model can be improved.

The affine transformation unit 63 in the first embodiment can arbitrarily set a rotation angle, an enlargement/reduction ratio, a parallel movement amount, and a shearing (skew) angle.

Then, each training image subjected to the affine transformation is stored in the training image storage unit 52 as a different training image by assigning an identification number or a name different from that of the training image before the affine transformation.

The trained model generation unit 64 performs machine training on a plurality of training images to generate a trained model. In the first embodiment, only an image (normal image) being confirmed in advance that no lesion is captured is used as the training image. In general, many images can be machine-trained because the accumulated images are more normal images that do not show lesions than abnormal images that show lesions.

Therefore, the trained model generation unit 64 generates a trained model using a machine training algorithm used for image abnormality detection, and can detect an abnormality in the image. For example, a machine training algorithm used for image abnormality detection such as a patch core performs machine training on a plurality of training images (normal images) as training data to generate a feature amount group (memory bank) of the normal images (normal data). Then, a plurality of trained models (candidate models) is generated by changing the number of times (the number of epochs) of repeatedly training one training image (training data). As a machine training algorithm used for image abnormality detection, an algorithm based on an AutoEncoding model, attention-guidance, SPADE, PaDiM, or the like can be used other than a patch core.

Furthermore, the trained model generation unit 64 may perform normalization in order to enhance the convergence speed of training and the stability of the training result. For example, as normalization, the pixel value is converted using an expression such as converted pixel value = (pixel value before conversion - average value)/(standard deviation). In addition, as the average value and the standard deviation, values used in training of the conventional method can also be used. In the first embodiment, the average value = [0.485, 0.456, 0.405] and the standard deviation = [0.229, 0.224, 0.225] are used.

The trained model determination unit 65 creates a trained model by training while confirming the trained model generated by the trained model generation unit 64 with the validation data, determines a model having a high correct answer rate in the validation data as a trained model (diagnosis model) used in the endoscope diagnosis program 1a, and stores the trained model (diagnosis model) in the trained model storage unit 54.

The trained model determination unit 65 according to the first embodiment inputs an image for validation in which the presence or absence of a lesion has been validated for each of a plurality of trained models (candidate models) generated by the trained model generation unit 64, and confirms whether or not a result obtained as an output matches the image for validation (whether or not it is correct). Then, the trained model (candidate model) having the highest correct answer rate of the confirmed resulting output is determined as a trained model (diagnosis model) used for endoscope diagnosis.

Next, each unit executed by the endoscope diagnosis program 1a will be described.

The diagnostic-use image acquisition unit 66 acquires a diagnostic-use image of a diagnosis target site captured with the endoscope device 20. The diagnostic-use image can be directly acquired from the endoscope device 20, or the diagnostic-use image stored in the diagnostic-use image storage unit 55 can be acquired.

The color tone correction unit 67 performs color tone correction on the diagnostic-use image (raw image) acquired from the endoscope device 20 or the diagnostic-use image storage unit 55, and stores the corrected diagnostic-use image (color tone corrected image) in the diagnostic-use image storage unit 55.

In the color tone correction executed here, the same color tone correction as the color tone correction of the training image used to generate the trained model is performed based on the reference color tone and the like. In this way, by performing the same color tone correction as the generation of the trained model on the diagnostic-use image, the image quality and color tone varying depending on the training image and the device type are unified, and the diagnosis accuracy is improved.

The lesion presence/absence diagnosis unit 68 inputs the corrected diagnostic-use image corrected by the color tone correction unit 67 to the trained model stored in the trained model storage unit 54, and diagnoses the presence or absence of a lesion based on the resulting output. In the first embodiment, when the feature amount of the diagnostic-use image is output to be 50% or more as the resulting output, the image is determined to be abnormal, it is diagnosed that there is a lesion, and the diagnosis result is output. On the other hand, when the feature amount of the diagnostic-use image is output to be less than 50% as the resulting output, the image is determined to be abnormal and diagnosed as having a lesion, and the diagnostic result is output.

Next, the operation of each configuration of the endoscope diagnosis device 1, the endoscope diagnosis program 1a, and the trained model generation program for endoscope diagnosis 10a of the first embodiment will be described together with an endoscope diagnosis device-control method.

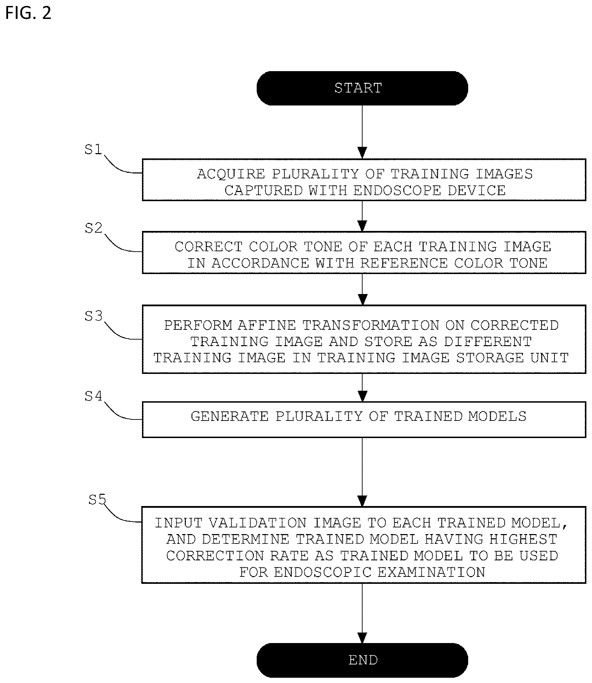

In the trained model generation program for endoscope diagnosis 10a, as illustrated in Fig. 2, the training image acquisition unit 61 acquires a plurality of training images (raw images) captured with the endoscope device stored in the training image storage unit 52 (training image acquisition step: S1). The training image (raw image) acquired at this time may include an image captured with an endoscope device of a different device type.

Next, the color tone correction unit 62 acquires the reference color tone stored in the reference color tone storage unit 53, and corrects the color tone of each training image in accordance with the reference color tone (color tone correcting step: S2). In a case where the training image includes an image captured with an endoscope device of a different device type, the color tone and the image quality of the training images are smoothed by this processing. The color tone correction unit 62 stores the corrected training image subjected to the color tone correction in the training image storage unit 52.

Next, the affine transformation unit 63 acquires the corrected training image stored in the training image storage unit 52, performs an arbitrary affine transformation, and stores the image in the training image storage unit 52 as a different training image (affine transformation step: S3). This allows for the pseudo-generation of images with varying shooting angles, as well as different distances and positions between the distal end of the endoscope camera and the diagnosis target site, while increasing the number of images to be machine-trained, without the need for actual image capture.

Next, the trained model generation unit 64 acquires a plurality of training images after correction and after affine transformation, and causes a machine training algorithm to train each acquired training image to generate a plurality of trained models (trained model generation step: S4). Specifically, a patch core, which is a machine training algorithm used for image abnormality detection, is caused to perform machine training using a plurality of training images as training data to generate a feature amount group of a normal image. Then, the number of times of repeatedly training one training image is changed to generate a plurality of trained models (candidate models).

Then, the trained model determination unit 65 inputs an image for validation in which the presence or absence of a lesion has been validated to the plurality of generated trained models, and determines the trained model having the highest correct answer rate as a trained model to be used for endoscope diagnosis (trained model determination step: S5). Specifically, for each trained model (candidate model) generated by the trained model generation unit 64, an image (abnormal image) in which a lesion is captured as an image for validation in which the presence or absence of a lesion has been validated, whether or not the image has been output as an abnormal image is confirmed, and the trained model (candidate model) having the highest correct answer rate output as an abnormal image is determined as a trained model (diagnostic model) used for endoscope diagnosis. Then, the trained model determined to be used for endoscope diagnosis is stored in the trained model storage unit 54.

This ends the execution of the trained model generation program for endoscope diagnosis 10a. The execution of the trained model generation program for endoscope diagnosis 10a is preferably ended in advance of the next diagnosis of the presence or absence of a lesion by the endoscope diagnosis program 1a.

Next, the presence or absence of a lesion is diagnosed by the endoscope diagnosis program 1a.

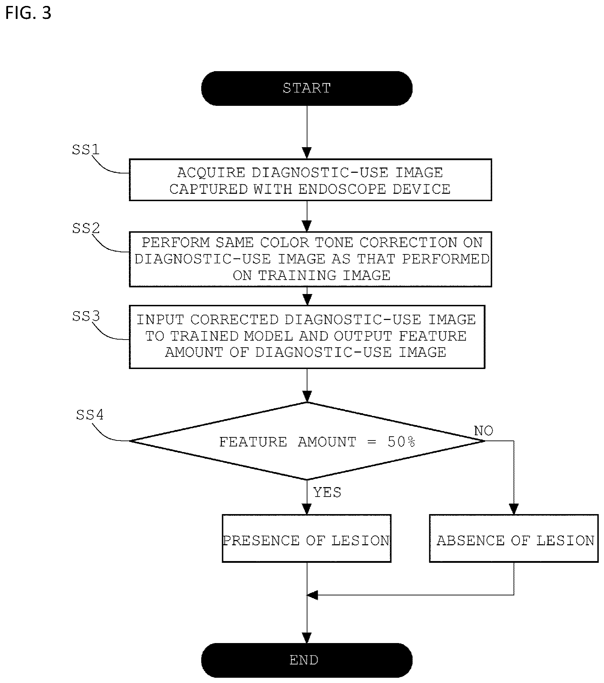

First, as illustrated in Fig. 3, the diagnostic-use image acquisition unit 66 acquires a diagnostic-use image (raw image) obtained by capturing a diagnosis target site from the endoscope device 20 or the diagnostic-use image storage unit 55 (diagnostic-use image acquisition step: SS1). The diagnostic-use image (raw image) acquired at this time may be provided by an endoscope device whose device type is different from the endoscope device that captured the training image used to generate the trained model.

Next, the color tone correction unit 67 acquires the reference color tone stored in the reference color tone storage unit 53, and performs the same color tone correction (including image quality enhancement processing) on the diagnostic-use image as that performed on the training image (color tone correction step: SS2). By performing the same color tone correction as that performed on the training image used to generate the trained model, the color tone and image quality are unified with those of the training image, and highly accurate diagnosis processing can be performed even on an image captured with the endoscope device 20 whose device type is different from the one that captured the training image.

Then, the lesion presence/absence diagnosis unit 68 inputs the corrected diagnostic-use image to the trained model stored in the trained model storage unit 54, and diagnoses the presence or absence of a lesion based on the resulting output (lesion presence/absence diagnosis step: SS3, SS4). Specifically, the corrected diagnostic-use image is input to the trained model, and the feature amount of the diagnostic-use image is output (SS3). Then, when the feature amount of the diagnostic-use image is output to be 50% or more as the resulting output (SS4: YES), the image is determined to be abnormal and diagnosed as having a lesion, and the result is output to the display means 2 or the like. On the other hand, when the feature amount of the diagnostic-use image is output to be less than 50% as a resulting output (SS4: NO), the image is determined to be normal and diagnosed as having no lesion, and the result is output to the display means 2 or the like.

This completes the diagnosis. The diagnosis result is not limited to that displayed on the display means 2, and may be externally output by an output unit such as a printer.

According to the first embodiment as described above, the following operational effects can be achieved.
1. By performing color tone correction and image quality enhancement processing on the training images accumulated so far, the color tone and image quality of the images can be smoothed, and the diagnosis accuracy based on the trained model can be improved.
2. By using the abnormality detection algorithm in the algorithm for generating the trained model, it is possible to use an image in which no lesion is captured as the training image. Therefore, it is possible to increase the number of images to be machine-trained, and to improve the diagnosis accuracy.
   Furthermore,
3. by performing affine transformation on the training image, it is possible to pseudo-generate images with varying shooting angles, as well as different distances and positions between the distal end of the endoscope camera and the diagnosis target site, without the need for actual image capture, and by storing those images as different images, it is possible to increase the number of images to be machine-trained and to enhance the diagnosis accuracy of the trained model.
4. By performing the same color tone correction and image quality enhancement processing on the diagnostic-use image as those performed on the training image, the color tone and image quality of the training image and the training image used to generate the trained model are unified, so that diagnosis accuracy based on the trained model can be improved.

Next, a second embodiment of an endoscope diagnosis program, an endoscope diagnosis device, an endoscope diagnosis device-control method, and a trained model generation program for endoscope diagnosis according to the present invention will be described with reference to the drawings. The same reference numerals are given to the same or corresponding configurations as those described in the aforementioned first embodiment, and their repeated description is omitted.

In the second embodiment, not only an image in which no lesion is captured in a training image but also an image in which a lesion is captured is used. By using the image in which a lesion is captured, the images accumulated so far can be more effectively utilized.

In the second embodiment, when a lesion is captured in a training image, the color tone correction unit 62 executes color tone correction in accordance with a reference color tone in an area other than the lesion existing area. By performing color tone correction separately for the lesion existing area (abnormal area) and the area other than the lesion existing area (normal area), it is possible to facilitate discrimination between the abnormal area and the normal area at the time of machine training, and by performing uniform color tone correction in accordance with the reference color tone for the normal area, it is possible to perform smoothing between images having different color tones and image quality, and to enhance the diagnosis accuracy of the generated trained model.

The area differentiation between the lesion existing area (abnormal area) and the area (normal area) other than the lesion existing area is performed by area differentiation using an existing program for automatically detecting a boundary in an image, manual area differentiation by pen input or the like. Although it takes more time and effort to differentiate the area than to perform color tone correction in the entire area, by which it becomes easy to distinguish between an abnormal area and a normal area, so that the accuracy of diagnosis can be improved.

In addition, in order to correspond to both images, the trained model generation unit 64 according to the second embodiment causes an algorithm that detects a feature amount of an image to perform machine training on the corrected training image (including the training image after affine transformation) to generate a trained model. Examples of algorithms for acquiring a feature amount from an image include,
an algorithm based on Scale-Invariant Feature Transform (SIFT), Speed Up Robust Feature (SURF), Oriented FAST and Rotated BRIDF (ORB), and Convolutional Neural Network (CNN), or the like.

Then, the lesion presence/absence diagnosis unit 68 according to the second embodiment inputs a diagnosis image to the generated trained model, diagnoses that there is a lesion when the feature amount of the diagnostic-use image is determined to be a predetermined value or more, and outputs the result to the display means 2 or the like. On the other hand, when the feature amount of the diagnostic-use image is determined to be less than the predetermined value, it is diagnosed that there is no lesion, and the result is output to the display means 2 or the like.

As a result, in the second embodiment, it is possible to diagnose the presence or absence of the lesion using the accumulated images regardless of the presence or absence of the captured lesion, and it is possible to more effectively utilize the images accumulated so far.

### [Example 1]

In Example 1, an endoscope diagnosis program according to the present invention was created, and the accuracy of diagnosis of the presence or absence of a lesion was validated using a diagnostic-use image (endoscope image) captured with an endoscope device.

### <Endoscope device>

The endoscope device used for the validation of Example 1 are three device types: CF-H290ECI manufactured by Olympus Corporation, CF-FH260AZI manufactured by Olympus Corporation, and EC-L600ZP7 manufactured by Fujifilm Corporation.

### <Diagnostic-use image>

The diagnostic-use image used for validation of Example 1 is an image obtained by capturing the inside of the large intestine as a diagnosis target site. Each image is classified in advance by a doctor into an image of a large intestine in a normal state in which a lesion such as inflammation or ulcer is not captured and is not congested (hereinafter, it is referred to as a "normal image") and an image of a large intestine in an abnormal state in which a lesion is captured and is congested (hereinafter, it is referred to as an "abnormal image").

Hereinafter, the diagnostic-use image captured with CF-H290ECI manufactured by Olympus Corporation is referred to as an "Olympus 290 image", the diagnostic-use image captured with CF-FH260AZI manufactured by Olympus Corporation is referred to as an "Olympus 260 image", and the diagnostic-use image captured with EC-L600ZP7 manufactured by Fujifilm Corporation is referred to as a "Fuji 600 image".

### <Software used for color tone correction>

In the validation of Example 1, Photoshop (registered trademark), which is image processing software manufactured by Adobe, was used to determine a reference color tone for correcting the color tone of the diagnostic-use image and to correct the color tone of the diagnostic-use image based on the reference color tone.

### <Determination of reference color tone (reference image)>

The reference color tone (reference image) in Example 1 was determined by the following procedure.

Procedure 1) Five images are randomly extracted from normal images among diagnostic-use images captured with one endoscope device.

Procedure 2) One image is selected as a representative image from the five diagnostic-use images extracted in Procedure 1.

Procedure 3) "Color correction" (Photoshop function) using the other four images as source images is executed on the representative image selected in Procedure 2.

Procedure 4) The representative image after executing Procedure 3 is determined as the reference color tone (reference image) and stored.

A method of selecting a representative image in Procedure 2 is arbitrary, but in Example 1, an image in which the lumen is captured most at the center is used as the representative image.

In addition, in Procedure 3, (1) the luminance, (2) the fitness of the color, and (3) the fade can be arbitrarily set within a range of 0 to 100. In Example 1, the parameters are set to (1): 100, (2): 100, and (3): 0 (default setting values), respectively.

In Example 1, the reference image determined using the "Olympus 290 image" was defined as an "Olympus reference image", and the reference image determined using the "Fuji 600 image" was defined as a "Fuji reference image".

### <Color tone correction based on reference color tone (creation of training image)>

The color tone correction in Example 1 is performed by executing "color correction" using the reference image as a source image on the diagnostic-use image. For example, when performing color tone correction in accordance with the "Olympus reference image" on the "Fuji 600 images", the "color correction" is performed on the "Fuji 600 images" using the "Olympus reference image" as the source image. At this time, (1) the luminance, (2) the fitness of color, and (3) the fade were set to (1): 100, (2): 100, and (3): 0 (default setting values), respectively, similarly to Procedure 3.

### <Generation of trained model>

In Example 1, a patch core, which is a machine training algorithm used for image abnormality detection, is used to generate a trained model. Specifically, the data set of the image to be validated was divided into a training image (training data) and test data, and machine training was performed by the patch core to generate a trained model.

However, the diagnostic accuracy of the generated trained model may vary depending on which data in the dataset is used as training images and which data is used as test data. Therefore, in Example 1, a method called stratified k-fold cross-validation is adopted.

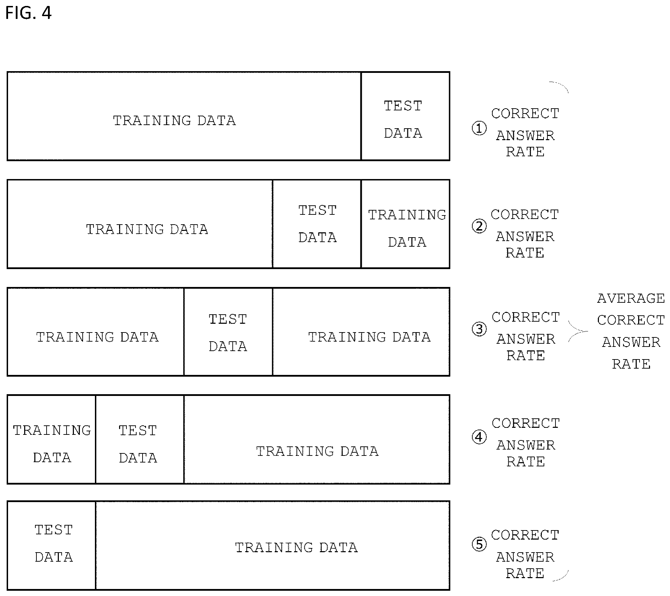

This stratified k-fold cross-validation, as shown in Fig. 4, involves dividing a dataset (image data group) into multiple image data groups. One of these groups is used as a diagnostic-use image, while the remaining groups are used as training images. For each fold, a trained model is generated, and its diagnostic accuracy is evaluated. The method calculates the average diagnostic accuracy across all the folds. Although it takes k times longer to validate, it is possible to suppress an error in the diagnosis accuracy by the division method. In Example 1, the stratified 5-fold cross-validation is performed with k = 5.

### <Diagnostic accuracy validation method>

In the diagnosis accuracy, a diagnostic-use image is input to the generated trained model to determine whether the diagnostic-use image is a normal image or an abnormal image. In Example 1, the diagnostic-use image (validation data: all of the training images and the test data used to generate the trained model) is determined by each of the five trained models generated by the stratified 5-fold cross-validation, and each correct answer rate and the average correct answer rate of the five correct answer rates are calculated.

Hereinafter, a validation result will be described.

### <Validation result by Olympus 290 image>

First, validation using an Olympus 290 image was performed. The total number of the Olympus 290 images used for validation is 287, which is 151 abnormal images and 136 normal images.

Table 1 below tabulates the results of the stratified 5-fold cross-validation using Olympus 290 images. In this table, the first from the left represents the division number, the second represents the loss function Loss based on the training data (the training image and the test data), the third represents the correct answer rate based on the training data, the fourth represents the loss coefficient Loss based on the diagnostic-use image (the validation data), and the fifth represents the correct answer rate of the diagnostic-use image.

**[Table 1]**

| Division number | Training data | | Validation data | |
|---|---|---|---|---|
| | Loss (↓) | Correct answer rate (↑) | Loss (↓) | Correct answer rate (↑) |
| 1 | 0.13350 | 0.95343 | 0.95203 | 0.72413 |
| 2 | 0.10870 | 0.96507 | 0.49783 | 0.81610 |
| 3 | 0.11647 | 0.96377 | 0.44907 | 0.78950 |
| 4 | 0.14077 | 0.95217 | 0.77340 | 0.82453 |
| 5 | 0.13667 | 0.95507 | 0.60573 | 0.80117 |

| | | | | |
|---|---|---|---|---|
| *Division number = Change division method of training and validation Average correct answer rate for validation data: 0.791 | | | | |

As shown in Table 1, the correct answer rate in the diagnostic-use image (validation data) for each division number was about 0.72 to about 0.82, and the average correct answer rate thereof was 0.791.

### <Validation result by Olympus 260 image>

Next, validation using the Olympus 260 image will be described. The total number of the Olympus 260 images used for validation is 239, which is 108 abnormal images and 131 normal images. The validation results are shown in the following Table 2.

**[Table 2]**

| Division number | Training data | | Validation data | |
|---|---|---|---|---|
| | Loss (↓) | Correct answer rate (↑) | Loss (↓) | Correct answer rate (↑) |
| 1 | 0.11223 | 0.95460 | 1.1596 | 0.61807 |
| 2 | 0.097933 | 0.96510 | 1.5428 | 0.58330 |
| 3 | 0.12117 | 0.95990 | 0.57780 | 0.77777 |
| 4 | 0.089600 | 0.97210 | 0.62347 | 0.73610 |
| 5 | 0.10553 | 0.96180 | 0.50683 | 0.75887 |

| | | | | |
|---|---|---|---|---|
| *Division number = Change division method of training and validation Average correct answer rate for validation data: 0.695 | | | | |

As shown in Table 2, the correct answer rate in the diagnostic-use image (validation data) for each division number was about 0.58 to about 0.77, and the average correct answer rate was 0.695. As compared with the case of the Olympus 290 image, the variation in the correct answer rate for each division number was large, and the average correct answer rate was also low.

### <Validation Result by Fuji 600 image>

Next, validation using the Fuji 600 image will be described. The total number of the Fuji 600 images used for validation is 232, which is 108 abnormal images and 124 normal images. The validation results are shown in the following Table 3.

**[Table 3]**

| Division number | Training data | | Validation data | |
|---|---|---|---|---|
| | Loss (↓) | Correct answer rate (↑) | Loss (↓) | Correct answer rate (↑) |
| 1 | 0.065500 | 0.98020 | 0.28897 | 0.87233 |
| 2 | 0.073233 | 0.97840 | 0.54157 | 0.80140 |
| 3 | 0.064667 | 0.96773 | 0.74487 | 0.78260 |
| 4 | 0.085900 | 0.97313 | 0.85803 | 0.65220 |
| 5 | 0.094533 | 0.96593 | 0.86723 | 0.71013 |

| | | | | |
|---|---|---|---|---|
| *Division number = Change division method of training and validation Average correct answer rate for validation data: 0.764 | | | | |

As shown in Table 3, the correct answer rate in the diagnostic-use image (validation data) for each division number was about 0.65 to about 0.87, and the average correct answer rate was 0.764. Although the variation in the correct answer rate for each division number is large as compared with the case of the Olympus 290 image, the average correct answer rate was 0.76, which was relatively high.

To summarize the above results, the average correct answer rate of the diagnosis results using the images captured with each device type was higher in the order of Olympus 290 image > Fuji 600 image > Olympus 260 image. In Example 1, the reference color tone (reference image) to be subjected to color tone correction was selected from those having a high average correct answer rate, and as described above, the "Olympus reference image" using the "Olympus 290 image" and the "Fuji reference image" using the "Fuji 600 image" were used.

### <Validation using Olympus 290 image and Olympus 260 image>

Next, a case where images captured with different types of endoscope devices are used together will be validated.

First, validation using an Olympus 290 image and an Olympus 260 image was performed. A data set obtained by adding the Olympus 290 images and the Olympus 260 images (without color tone correction) was used to perform stratified 5-fold cross-validation.

The number of the Olympus 290 images used for validation is 152 abnormal images and 137 normal images, that is, 289 in total, and the number of the Olympus 260 images is 109 abnormal images and 133 normal images, that is, 242 in total. Therefore, when the both images were added together, the total is 531, which is 261 abnormal images and 270 normal images.

Results of validation based on the data set obtained by adding the Olympus 290 images and the Olympus 260 images (without color tone correction) are shown in Table 4 below.

**[Table 4]**

| Division number | Training data | | Validation data | |
|---|---|---|---|---|
| | Loss (↓) | Correct answer rate (↑) | Loss (↓) | Correct answer rate (↑) |
| 1 | 0.13007 | 0.95480 | 0.58777 | 0.83333 |
| 2 | 0.15537 | 0.93823 | 0.43140 | 0.86030 |
| 3 | 0.18453 | 0.93110 | 0.57810 | 0.82540 |
| 4 | 0.16700 | 0.94697 | 0.65870 | 0.74603 |
| 5 | 0.14287 | 0.94613 | 0.46987 | 0.80000 |

| | | | | |
|---|---|---|---|---|
| Average correct answer rate for validation data: 0.813 | | | | |

As shown in Table 4, the correct answer rate in the diagnostic-use image (validation data) for each division number was about 0.74 to about 0.86, and the average correct answer rate was 0.813.

As compared with the average correct answer rate of 0.791 when the Olympus 290 images were independently validated, the average correct answer rate was 0.813, and the accuracy was improved. This is considered to be because the number of data items to be machine-trained has approximately doubled compared to the case where the Olympus 290 images are independently validated, and thus the accuracy of the trained model is improved. On the other hand, the correct answer rate in the diagnostic-use image (validation data) for each division number was about 0.74 to about 0.86, and there was a slight variation.

### <Validation using Olympus 290 image and color-tone-corrected Olympus 260 image>

Next, in order to confirm the effectiveness of the color tone correction, stratified 5-fold cross-validation was performed using a data set obtained by adding the Olympus 290 image and the Olympus 290 image whose color tone has been corrected in accordance with the color tone of the Olympus 260 image.

The number of images of the Olympus 290 images and the Olympus 260 images used for validation is the same as for the validation based on the results of Table 4. On the other hand, in order to match the color tone of the Olympus 260 image with the color tone of the Olympus 290 image, color tone correction using the Olympus reference image as the source image was performed on all the images.

The validation results based on the data set obtained by adding the Olympus 290 images and the color-tone-corrected Olympus 260 images are shown in Table 5 below.

**[Table 5]**

| Division number | Training data | | Validation data | |
|---|---|---|---|---|
| | Loss (↓) | Correct answer rate (↑) | Loss (↓) | Correct answer rate (↑) |
| 1 | 0.13357 | 0.94773 | 0.74020 | 0.82390 |
| 2 | 0.15270 | 0.93743 | 0.60337 | 0.82080 |
| 3 | 0.16573 | 0.93127 | 0.41667 | 0.84443 |
| 4 | 0.14570 | 0.94943 | 0.51350 | 0.81903 |
| 5 | 0.13075 | 0.94550 | 0.69370 | 0.82537 |

| | | | | |
|---|---|---|---|---|
| Average correct answer rate for validation data: 0.827 | | | | |

As shown in Table 5, the correct answer rate in the diagnostic-use image (validation data) for each division number was about 0.81 to about 0.84, and there was little variation in the correct answer rate. It is considered that by adjusting the color tone by the color tone correction, a uniform trained model can be generated regardless of the data used for training.

Furthermore, the average correct answer rate was 0.827, and the accuracy was improved as compared with the average correct answer rate of 0.813 in a case where the color tone of the Olympus 260 image was not corrected.

Therefore, in a case where the endoscope images captured with different types of endoscope devices are used together, by performing the color tone correction to match the color tone with either model, it is possible to confirm the improvement of the diagnosis accuracy as compared with the case where the color tone correction is not performed.

### <Validation using Fuji 600 image, Olympus 290 image (without color tone correction) and Olympus 260 image (without color tone correction)>

Next, validation was performed by a combination of endoscope images captured with endoscope devices of different manufacturers.

The Fuji 600 images used in the validation are a total of 234 images including 109 abnormal images and 125 normal images, the Olympus 290 image is a total of 289 images including 152 abnormal images and 137 normal images, and the Olympus 260 image is a total of 242 images including 109 abnormal images and 133 normal images. Therefore, when all the images were added up, the total is 765, which is 370 abnormal images and 395 normal images.

A data set obtained by adding the Fuji 600 images, the Olympus 290 images (without color tone correction), and the Olympus 260 images (without color tone correction) was used to perform stratified 5-fold cross-validation. The validation results are shown in Table 6 below.

**[Table 6]**

| Division number | Training data | | Validation data | |
|---|---|---|---|---|
| | Loss (↓) | Correct answer rate (↑) | Loss (↓) | Correct answer rate (↑) |
| 1 | 0.14117 | 0.95050 | 0.64387 | 0.80923 |
| 2 | 0.19450 | 0.92297 | 0.40080 | 0.83333 |
| 3 | 0.15547 | 0.93950 | 0.60360 | 0.81797 |
| 4 | 0.13580 | 0.94457 | 0.77213 | 0.79910 |
| 5 | 0.15470 | 0.93850 | 0.56370 | 0.81680 |

| | | | | |
|---|---|---|---|---|
| Average correct answer rate for validation data: 0.815 | | | | |

As shown in Table 6, the average correct answer rate by the diagnostic-use image (validation data) was 0.815, and the accuracy was improved as compared with the average correct answer rate 0.764 in the case of using only the Fuji 600 image. Regarding this improvement in accuracy, it is considered that the accuracy of the trained model has improved since the number of data to be machine-trained has approximately tripled, similarly to the case of adding the Olympus 290 images and the Olympus 260 images (without color tone correction).

### <Validation using Fuji 600 image, color-tone-corrected Olympus 290 image, and color-tone-corrected Olympus 260 image>

Next, in order to confirm the effectiveness of the color tone correction, stratified 5-fold cross-validation was performed using a data set obtained by adding the Fuji 600 image, the tone-corrected Olympus 290 image, and the tone-corrected Olympus 260 image. The validation results are shown in Table 7 below.

**[Table 7]**

| Division number | Training data | | Validation data | |
|---|---|---|---|---|
| | Loss (↓) | Correct answer rate (↑) | Loss (↓) | Correct answer rate (↑) |
| 1 | 0.17533 | 0.92610 | 0.66180 | 0.79303 |
| 2 | 0.15787 | 0.93543 | 0.62607 | 0.86493 |
| 3 | 0.18507 | 0.92403 | 0.92150 | 0.75657 |
| 4 | 0.16740 | 0.93223 | 0.61123 | 0.82237 |
| 5 | 0.16830 | 0.93440 | 0.49693 | 0.86620 |

| | | | | |
|---|---|---|---|---|
| Average correct answer rate for validation data: 0.821 | | | | |

As shown in Table 7, the average correct answer rate by the diagnostic-use image (validation data) was 0.821, and the accuracy was improved as compared with the average correct answer rate of 0.764 in the case of using only the Fuji 600 image and the average correct answer rate of 0.815 in the case of not performing color tone correction.

### <Validation using Fuji 600 image and color-tone corrected Olympus 290 image>

In addition, stratified 5-fold cross-validation was also performed using, as a data set, a combination of the Fuji 600 images and the Olympus 290 images subjected to color tone correction based on the Fuji reference images. The validation results are shown in Table 8 below.

**[Table 8]**

| Division number | Training data | | Validation data | |
|---|---|---|---|---|
| | Loss (↓) | Correct answer rate (↑) | Loss (↓) | Correct answer rate (↑) |
| 1 | 0.16417 | 0.93777 | 0.45257 | 0.82853 |
| 2 | 0.12627 | 0.95373 | 0.34990 | 0.88890 |
| 3 | 0.16400 | 0.94340 | 0.52740 | 0.80317 |
| 4 | 0.087267 | 0.97133 | 0.50990 | 0.83653 |
| 5 | 0.13887 | 0.94510 | 0.54200 | 0.85580 |

| | | | | |
|---|---|---|---|---|
| Average correct answer rate for validation data: 0.843 | | | | |

As shown in Table 8, all the correct answer rates in the diagnostic-use images (validation data) for each division number were 0.8 or more, and the average correct answer rate was also 0.843, which was a high correct answer rate.

### <Validation using Fuji 600 image and color-tone corrected Olympus 260 image>

Furthermore, stratified 5-fold cross-validation was also performed using, as a data set, a combination of the Fuji 600 images and the Olympus 260 images subjected to color tone correction based on the Fuji reference images. As shown in Table 9 below, the average correct answer rate was as high as 0.845, and a high correct answer rate was obtained.

**[Table 9]**

| Division number | Training data | | Validation data | |
|---|---|---|---|---|
| | Loss (↓) | Correct answer rate (↑) | Loss (↓) | Correct answer rate (↑) |
| 1 | 0.076933 | 0.97427 | 1.0317 | 0.83160 |
| 2 | 0.13130 | 0.95227 | 0.34423 | 0.88297 |
| 3 | 0.12580 | 0.95490 | 0.54113 | 0.82623 |
| 4 | 0.11910 | 0.95317 | 0.70587 | 0.79433 |
| 5 | 0.10923 | 0.96463 | 0.33790 | 0.89007 |

| | | | | |
|---|---|---|---|---|
| Average correct answer rate for validation data: 0.845 | | | | |

As described above, according to the present invention, it has been confirmed that the color tone between the images is smoothed by performing color tone correction on the diagnostic-use image and the training images accumulated so far, and the diagnosis accuracy based on the trained model can be improved regardless of the manufacturer and the device type of the endoscope device.

### <Validation of trained model using only normal image>

It is conceivable that the number of normal images in which no lesion is captured increases in the images that can be collected as training images for machine training. Therefore, the trained model generated using only the normal image for the training data was validated.

The number of images used for validation is 115 normal images of the Fuji 600 images, 131 normal images of the Olympus 290 images, and 125 normal images of the Olympus 260 image, that is, 371 in total.

Here, (1) the accuracy of the trained model in the case of machine training of the data set obtained by adding the normal images of the Fuji 600 images, the Olympus 290 images, and the Olympus 260 images, (2) the accuracy of the trained model in the case of machine training of the data set obtained by adding the normal images of the Fuji 600 images, the color-tone corrected Olympus 290 images, and the color-tone corrected Olympus 260 images, (3) the accuracy of the trained model in the case of machine training of only the normal images of the Fuji 600 images, (4) the accuracy of the trained model in the case of machine training of only the normal images of the Olympus 260 images, and (5) the accuracy of the trained model in the case of machine training of only the normal images of the Olympus 290 images were validated.

As an index for evaluating validation of accuracy, Area Under the ROC Curve (AUROC), which is one of evaluation indexes for a binary classification problem in machine training, and an F value (F-measure), which is one of evaluation indexes for a binary classification problem and is a harmonic average of a precision and a recall, were calculated together with the average correct answer rate. AUROC takes a value in a range of 0 to 1, and the closer to 1, the higher the prediction performance of the model. In addition, the F value takes a value in a range of 0 to 1, and as the F value is closer to 1, it indicates that the machine trained model is a well-balanced machine trained model capable of achieving both the precision and the recall. The respective validation results are shown in the following Table 10.

**[Table 10]**

| Name of device type | AUROC | F1Score | Correct answer rate |
|---|---|---|---|
| All | 0.5703 | 0.5778 | 0.5250 |
| Fuji color-tone-correction | 0.6484 | 0.7619 | 0.6250 |
| Fuj i | 0.5157 | 0.6316 | 0.5625 |
| Olympus 260 | 0.5556 | 0.7500 | 0.6000 |
| Olympus 290 | 0.5481 | 0.7273 | 0.6538 |

As shown in Table 10, higher values were obtained in all of the AUROC, F value, and average correct answer rate in the case of performing color tone correction in accordance with the Fuji reference image of (2) than in the case of adding all the normal images of (1). With respect to the average correct answer rate in the trained model of the case where machine training was performed on only the normal images of the Olympus 290 images of (5), a result was obtained in which the accuracy was higher than the result of the case where the color tone correction of (2) was performed, but in other cases, a result was obtained in which the accuracy was high in the case where the color tone correction of (2) was performed.

As described above, it has been confirmed that the accuracy is improved by matching the color tone by the color tone correction even for the trained model obtained through machine training only the normal images in the endoscope images captured with different device types and manufacturers of endoscope devices. Therefore, it has been confirmed that even if more normal images in which no lesion is captured are collected as training images in the future, the accuracy of the trained model used to diagnose the presence or absence of lesions can be improved.

Note that the endoscope diagnosis program, the endoscope diagnosis device, the endoscope diagnosis device-control method, and the trained model generation program for endoscope diagnosis according to the present invention are not limited to the above-described embodiments, and can be appropriately changed. For example, the trained model generation unit 64 may further improve the diagnosis accuracy of the trained model generated by performing ensemble training. Furthermore, in a case where the image sizes of the training image and the diagnostic-use image are different, the color tone correction units 62 and 67 may execute processing of adjusting the images to a uniform size.

### REFERENCE SIGNS LIST

- 1: Endoscope diagnosis device
- 1a: Endoscope diagnosis program
- 2: Display means
- 3: Input means
- 4: Computer
- 5: Storage means
- 6: Arithmetic processing means
- 10a: Trained model generation program for endoscope diagnosis
- 20: Endoscope device
- 21: Endoscope camera
- 51: Program storage unit
- 52: Training image storage unit
- 53: Reference color tone storage unit
- 54: Trained model storage unit
- 55: Diagnostic-use image storage unit
- 61: Training image acquisition unit
- 62: Color tone correction unit
- 63: Affine transformation unit
- 64: Trained model generation unit
- 65: Trained model determination unit
- 66: Diagnostic-use image acquisition unit
- 67: Color tone correction unit
- 68: Lesion presence/absence diagnosis unit

## Claims

1. An endoscope diagnosis program for diagnosing presence or absence of a lesion based on an endoscope image captured with an endoscope device, the endoscope diagnosis program causing a computer to function as:
a diagnostic-use image acquisition unit that acquires a diagnostic-use image of a diagnosis target site captured with an endoscope device;
a color tone correction unit that performs color tone correction on the diagnostic-use image in accordance with a reference color tone that is a color tone of an image of a diagnosis target site captured in advance with the endoscope device; and
a lesion presence/absence diagnosis unit that inputs the corrected diagnostic-use image into a trained model and that performs diagnosis processing of the presence or absence of a lesion based on a resulting output, the trained model having been generated through machine training of a plurality of training images that have been subjected to color tone correction in accordance with the reference color tone.

2. The endoscope diagnosis program according to claim 1, wherein
the color tone correction unit performs the color tone correction and image quality enhancement processing on the diagnostic-use image, and
the lesion presence/absence diagnosis unit performs diagnosis processing of the presence or absence of a lesion by the trained model that has been generated through machine training of the training images that have undergone the same color correction and image quality enhancement processing as performed by the color correction unit.

3. The endoscope diagnosis program according to claim 1, wherein the lesion presence/absence diagnosis unit performs, as an image abnormality detection algorithm, inputting the corrected diagnostic-use image into a trained model that has been generated through machine training of a plurality of the training images corrected in accordance with the reference color tone and in which no lesion is captured, and diagnosing the presence of a lesion when an abnormality in the diagnostic-use image is detected as a resulting output.

4. The endoscope diagnosis program according to claim 1, wherein the lesion presence/absence diagnosis unit performs, as an algorithm for detecting an image feature amount, inputting the corrected diagnostic-use image into a trained model that has been generated through machine training of a plurality of the training images corrected in accordance with the reference color tone and in which no lesion is captured and a plurality of the training images corrected in accordance with the reference color tone and in which lesions are captured, and diagnosing the presence of a lesion when the feature amount of the diagnostic-use image is determined to be a predetermined value or more as a resulting output, and diagnosing the absence of a lesion when the feature amount of the diagnostic-use image is determined to be less than the predetermined value as a resulting output.

5. An endoscope diagnosis device for diagnosing presence or absence of a lesion based on an endoscope image captured with an endoscope device, the endoscope diagnosis device comprising:
a diagnostic-use image acquisition unit that acquires a diagnostic-use image of a diagnosis target site captured with an endoscope device;
a color tone correction unit that performs color tone correction on the diagnostic-use image in accordance with a reference color tone that is a color tone of an image of a diagnosis target site captured in advance with the endoscope device; and
a lesion presence/absence diagnosis unit that inputs the corrected diagnostic-use image into a trained model and that performs diagnosis processing of the presence or absence of a lesion based on a resulting output, the trained model having been generated through machine training of a plurality of training images that have been subjected to color tone correction in accordance with the reference color tone.

6. An endoscope diagnosis device-control method for diagnosing presence or absence of a lesion based on an endoscope image captured with an endoscope device, the endoscope diagnosis device-control method comprising:
a diagnostic-use image acquisition step of acquiring a diagnostic-use image of a diagnosis target site captured with an endoscope device;
a color tone correction step of performing color tone correction on the diagnostic-use image in accordance with a reference color tone that is a color tone of an image of a diagnosis target site captured in advance with the endoscope device; and
a lesion presence/absence diagnosis step of inputting the corrected diagnostic-use image into a trained model and performing diagnosis processing of the presence or absence of a lesion based on a resulting output, the trained model having been generated through machine training of a plurality of training images that have been subjected to color tone correction in accordance with the reference color tone.

7. A trained model generation program for endoscope diagnosis for generating a trained model used in endoscope diagnosis for diagnosing presence or absence of a lesion based on an endoscope image captured with an endoscope device, the trained model generation program causing a computer to function as:
a training image acquisition unit that acquires a plurality of training images of a diagnosis target site captured with an endoscope device;
a color tone correction unit that performs color tone correction on each of the training images in accordance with a reference color tone that is a color tone of an image of a diagnosis target site captured in advance with the endoscope device;
a trained model generation unit that generates a plurality of trained models using a machine training algorithm; and
a trained model determination unit that inputs a validation image in which presence or absence of a lesion has been validated to each of the generated trained models and that determines the trained model having a highest correct answer rate as a trained model to be used for endoscope diagnosis.

8. The trained model generation program for endoscope diagnosis according to claim 7, wherein in a case where a lesion is captured in the training image, the color tone correction unit executes color tone correction in accordance with the reference color tone in an area other than a lesion existing area.

9. The trained model generation program for endoscope diagnosis according to claim 7, wherein
the trained model generation program causes a computer to function as an affine transformation unit that performs an arbitrary affine transformation on the training image corrected by the color tone correction unit, and
the trained model generation unit causes machine training to be performed on the training image transformed by the affine transformation unit together with the training image corrected by the color tone correction unit to generate a trained model.
